# FASCICULE DE BREVET EUROPEEN

(11) **EP 4 656 929 B1**
(45) Date de publication et mention de la délivrance du brevet: **11.11.2026**
(21) Numéro de dépôt: 25160588.7
(22) Date de dépôt: 27.02.2025
(51) Int. Cl.: F16N 19/00, F16N 21/06, F01M 11/04

(54) **SYSTÈME MÉCANIQUE MUNI D'UN DISPOSITIF DE PRÉLÈVEMENT D'HUILE**
MECHANISCHES SYSTEM MIT ÖLENTNAHMEVORRICHTUNG
MECHANICAL SYSTEM WITH OIL REMOVAL DEVICE

(30) Priorité: 31.05.2024 FR 2405665
(43) Date de publication de la demande: 03.12.2025
(73) Titulaire: Airbus Helicopters, 13725 Marignane Cedex (FR)
(72) Inventeur: FABRIGAT, Eric, 13880 VELAUX (FR)
(74) Mandataire: GPI Brevets

(56) Documents cités:
- CN-U- 217 717 083
- CN-U- 218 180 400
- JP-A- 2017 110 667
- US-A1- 2018 355 987

## Description

La présente invention concerne un système mécanique muni d'un dispositif de prélèvement d'huile. Par exemple, un tel système mécanique peut être une boîte de transmission de puissance d'un aéronef, et en particulier peut être une boîte de transmission de puissance mise en mouvement par au moins un moteur pour entrainer en rotation au moins un rotor d'un giravion.

A titre illustratif, un système mécanique peut comprendre des éléments mobiles lubrifiés et/ou refroidis avec de l'huile. Le système mécanique comporte alors une prise de remplissage afin de permettre d'introduire l'huile dans un volume interne du système mécanique. En dehors de cette phase de remplissage, la prise de remplissage peut être obturée par un bouchon. En outre, une crépine amovible peut être disposée dans la prise de remplissage pour limiter les risques d'introduction de polluants dans le volume interne du système mécanique conjointement avec l'huile.

Des procédures de maintenance peuvent prévoir de prélever une partie de l'huile à des fins d'analyse, par exemple afin de détecter un écaillage. Dès lors, un opérateur retire le bouchon et éventuellement la crépine, puis introduit un tuyau de prélèvement dans le système mécanique, via la prise de remplissage, afin de prélever une partie de l'huile.

Bien qu'une telle procédure soit efficace, des polluants peuvent éventuellement être introduits par accident dans le système mécanique lors de cette opération de maintenance. En outre, il peut être difficile de déterminer précisément où l'huile a été prélevée dans le système mécanique, ce qui peut éventuellement compliquer l'analyse des résultats.

Par ailleurs, on connait des presse-étoupes et des dispositifs porte-serviettes qui n'appartiennent pas au domaine technique des dispositifs de maintenance d'un système mécanique.

La présente invention a alors pour objet de proposer un système mécanique innovant permettant de prélever de l'huile à des fins de maintenance en limitant les risques de pollution.

Les documents CN 218180400 U, CN 217717083 U, JP 2017110667 A, et US 20180355987 A1 sont connus.

En particulier, le document CN 218180400 U décrit un simple tuyau passant au travers d'une fixation à visser, le tuyau comprenant un contrepoids.

L'invention vise ainsi un système mécanique muni d'une enveloppe délimitant un volume interne contenant de l'huile.

Ce système mécanique comporte un dispositif de prélèvement pour prélever au moins une partie de l'huile, le dispositif de prélèvement comprenant au moins une tuyauterie solidaire de l'enveloppe, la tuyauterie s'étendant d'une extrémité interne située dans le volume interne jusqu'à un col comprenant une extrémité externe ouverte sur un milieu extérieur situé à l'extérieur du système mécanique, le dispositif de prélèvement comprenant un obturateur manœuvrable par un opérateur pour obturer le col, le dispositif de prélèvement comprenant dans le col une barrière anti-polluant ménageant un passage, voire un unique passage, restreint, apte à être traversé par un tuyau de prélèvement.

Le tuyau de prélèvement peut faire partie du dispositif de prélèvement. En particulier, le tuyau de prélèvement peut faire partie d'un kit de prélèvement comprenant de plus une pompe d'aspiration apte à aspirer l'huile dans le tuyau de prélèvement et/ou un récipient apte à recueillir l'huile aspirée par le tuyau de prélèvement.

Le terme « tuyauterie » désigne une liaison fluidique pouvant comprendre un ou des tuyaux, un ou des conduits structurels de l'enveloppe, et/ou un ou des connecteurs hydrauliques. Par exemple, la tuyauterie peut comprendre un conduit qui est ménagé dans l'enveloppe, et une prise de prélèvement qui est fixée à l'enveloppe de manière à déboucher sur le conduit et à former le col. Selon un autre exemple, la tuyauterie comporte un conduit rigide fixé à l'enveloppe par de moyens usuels de vissage, de collage, de soudure, de rivetage ou autres.

Dès lors, le système mécanique comporte une tuyauterie obturée par l'obturateur au repos, à savoir en dehors d'une phase de prélèvement d'huile. Lors d'une telle phase de prélèvement d'huile, un opérateur manœuvre l'obturateur pour libérer l'accès vers le volume interne. La barrière anti-polluant limite alors les risques d'introduire des polluants dans le volume interne.

En effet, la barrière anti-polluant restreint une surface de passage de la tuyauterie à un passage restreint. L'expression « passage restreint » signifie que le passage présente une aire inférieure à l'aire de la section de la tuyauterie logeant la barrière anti-polluant. En effet, cette section a au repos une aire égale à l'aire du passage restreint et à l'aire couverte par la barrière anti-polluant, voire à l'aire d'un organe solidarisant la barrière anti-polluant à la tuyauterie. L'opérateur introduit ensuite le tuyau de prélèvement dans la tuyauterie en le faisant traverser la barrière anti-pollution par le passage restreint. De l'huile peut alors être extraite par ce tuyau de prélèvement à des fins d'analyse. L'opérateur peut ensuite retirer le tuyau de prélèvement et repositionner l'obturateur.

Un tel dispositif de prélèvement limite donc en utilisation les risques de pollution accidentelle de l'huile dans le volume interne, en raison de la barrière anti-pollution agencée dans la tuyauterie entre le tuyau de prélèvement et cette tuyauterie. Au repos, l'obturateur protège en outre ce volume interne en obturant totalement la tuyauterie.

En outre, la tuyauterie sert aussi de guide en guidant le déplacement du tuyau de prélèvement dans le volume interne. Afin de réaliser un prélèvement d'huile dans une zone précise, l'extrémité interne de la tuyauterie peut être localisée dans la zone recherchée. Cette zone peut être située sensiblement à mi-hauteur du volume d'huile au sein de l'enveloppe afin que l'huile prélevée soit représentative de l'huile circulant dans le système mécanique, en étant par exemple dépourvue des particules lourdes qui reposent au fond de l'enveloppe. Alternativement, cette zone peut être choisie pour détecter toutes sortes de polluants, par exemple de l'eau ou autres dans des localisations particulières du système mécanique. Le système mécanique peut en outre comprendre plusieurs tuyauteries pour pouvoir sonder plusieurs zones et/ou des tuyaux de prélèvement ayant des longueurs différentes peuvent être introduits dans une même tuyauterie à cet effet.

La tuyauterie peut être en outre plus rigide que le tuyau de prélèvement, la rigidité de la tuyauterie permettant à cette tuyauterie de ne pas se déformer lors de l'insertion du tuyau de prélèvement. La souplesse du tuyau de prélèvement permet au contraire de l'insérer et le déplacer facilement dans la tuyauterie.

Par suite, le dispositif de prélèvement selon l'invention s'avère relativement simple et permet de réaliser un prélèvement précis d'huile au sein du système mécanique, en limitant les risques d'introduire des polluants, en particulier de la poussière, dans le système mécanique lors d'une telle opération.

Le système mécanique peut de plus comporter une ou plusieurs des caractéristiques qui suivent, prises seules ou en combinaison.

Selon une possibilité, la barrière anti-polluant peut être disposée dans la tuyauterie entre l'extrémité externe et l'extrémité interne.

La barrière anti-polluant n'est alors pas située dans l'extrémité externe. Une telle caractéristique limite par exemple les risques de dégrader cette barrière anti-polluant en utilisation.

Selon une possibilité compatible avec les précédentes, ladite barrière anti-polluant peut être amovible.

La barrière anti-polluant peut, à titre illustratif, être disposée sur un anneau inséré dans la tuyauterie. Cet anneau peut par exemple être vissé à la tuyauterie, peut comprendre des pattes glissant dans des gorges de la tuyauterie, peut comprendre une butée reposant sur l'extrémité externe ou autres.

Indépendamment de la variante, la barrière anti-polluant peut être connectée à la tuyauterie via un système réversible. Cette barrière anti-polluant peut alors être facilement remplacée en présence d'une détérioration.

Alternativement, la barrière anti-polluant peut être fixée par des moyens usuels à la tuyauterie.

Eventuellement, une grille peut être disposée dans la tuyauterie en aval de la barrière anti-polluant, selon un sens allant vers le milieu interne, pour éviter l'introduction d'une partie de cette barrière anti-polluant dans la tuyauterie en cas de détérioration.

Selon une possibilité compatible avec les précédentes, la barrière anti-polluant peut comporter une membrane élastique délimitant ledit passage restreint.

La membrane élastique peut comprendre un orifice formant le passage restreint précité. Par exemple, la membrane élastique est trouée pour former le passage, éventuellement en son centre. Au regard d'un axe passant à l'intérieur du col, la membrane élastique peut s'étendre radialement de la paroi interne de la tuyauterie jusqu'au passage. La membrane peut ainsi comprendre une section externe annulaire sensiblement accolée à la paroi interne et une section interne trouée pour former le passage. Le passage peut avoir favorablement une forme circulaire, ou alternativement une forme en croix, une forme en étoile, etc.

Une telle membrane élastique peut délimiter un passage plus petit que le tuyau de prélèvement.

Dès lors, les risques d'introduction de polluants sont réduits.

En outre, la membrane élastique peut permettre de maintenir en position le tuyau de prélèvement lors du prélèvement d'huile.

Selon une possibilité, le dispositif de prélèvement comportant le tuyau de prélèvement, la membrane élastique peut s'étirer lorsque le tuyau de prélèvement est introduit en élargissant ledit passage, la membrane élastique épousant le tuyau de prélèvement.

Lorsque le tuyau de prélèvement est inséré, ce tuyau de prélèvement exerce un effort sur la membrane élastique. La membrane élastique se déforme sans se déchirer ce qui permet d'augmenter les dimensions du passage jusqu'à permettre au tuyau de prélèvement de traverser le passage, ce passage pouvant être conformé au tuyau de prélèvement.

Dès lors, le passage peut être de très petites dimensions au repos, et peut être grandement, voire complétement, rempli par le tuyau de prélèvement en utilisation. Les risques d'introduction de polluants sont réduits.

Eventuellement, la membrane élastique peut comporter une matière du groupe des élastomères, voire en particulier du groupe des caoutchoucs.

Une telle matière permet d'obtenir une membrane suffisamment rigide au repos pour faire obstacle à des polluants, et suffisamment souple pour pouvoir être déformée et traversée par le tuyau de prélèvement en utilisation.

Selon une possibilité compatible avec les précédentes, le dispositif de prélèvement pouvant comporter le tuyau de prélèvement précédemment décrit, le tuyau de prélèvement peut être plus fin, à savoir plus étroit, que la tuyauterie pour pouvoir être introduit dans la tuyauterie et passer au travers de ladite barrière anti-polluant via le passage.

Cette caractéristique permet d'introduire le tuyau de prélèvement dans la tuyauterie.

En outre, la tuyauterie peut avoir un diamètre interne plus important que le diamètre externe du tuyau de prélèvement, mais plus petit que le diamètre d'une prise de remplissage conventionnelle.

Selon une possibilité compatible avec les précédentes, le dispositif de prélèvement pouvant comporter le tuyau de prélèvement précédemment décrit, le tuyau de prélèvement peut avoir une longueur calibrée par rapport à une longueur de la tuyauterie afin de sortir de part et d'autre de la tuyauterie et en particulier en dehors de la tuyauterie du côté du milieu extérieur d'une longueur de dépassement prédéterminée durant une phase de prélèvement d'huile.

Dès lors, le dispositif de prélèvement permet de positionner de manière reproductible le tuyau de prélèvement dans le volume interne. Le tuyau de prélèvement dépasse de la tuyauterie d'une longueur déterminée, sensiblement identique à chaque prélèvement, pour atteindre la zone de prélèvement ciblée. A titre illustratif, la tuyauterie peut avoir une longueur de 30 centimètres, et le tuyau de prélèvement peut avoir une longueur de 50 centimètres. En indiquant que le tuyau de prélèvement doit dépasser de 15 centimètres dans le milieu extérieur, le dispositif de prélèvement garantit que le tuyau de prélèvement dépassera sensiblement de la tuyauterie dans le volume interne d'une longueur de 5 centimètres. L'extrémité du tuyau de prélèvement présente dans le volume interne sera ainsi sensiblement toujours à la même place.

Selon une possibilité compatible avec les précédentes, le dispositif de prélèvement pouvant comporter le tuyau de prélèvement précédemment décrit, le tuyau de prélèvement peut comporter au moins un marquage ou une protubérance configuré pour placer le tuyau de prélèvement à une position prédéterminée par rapport à la tuyauterie, en particulier par rapport à l'extrémité externe ouverte de la tuyauterie.

Ce marquage ou cette protubérance peuvent être positionnés sensiblement au niveau de l'extrémité externe de la tuyauterie lors de l'insertion du tuyau de prélèvement. Ce marquage ou cette protubérance permettent de placer précisément et de façon reproductible le tuyau de prélèvement.

Selon une possibilité compatible avec les précédentes, l'obturateur peut comporter un opercule articulé au col ou vissé au col pour obturer ce col.

Un tel opercule peut obturer efficacement l'extrémité externe.

L'opercule et/ou le col peuvent comprendre un moyen d'étanchéité, tel qu'un joint par exemple, afin que l'opercule puisse obturer la tuyauterie de manière étanche à l'huile par exemple.

De manière complémentaire ou alternative, l'obturateur peut comporter un bouchon vissé à une paroi externe du col.

Un bouchon conventionnel peut obturer le col.

Le bouchon et/ou le col peuvent comprendre un organe d'étanchéité, tel qu'un joint par exemple, afin que le bouchon puisse obturer la tuyauterie de manière étanche à l'huile par exemple.

Eventuellement, l'obturateur comporte le bouchon et l'opercule précités. Le bouchon peut recouvrir l'opercule quand ce bouchon est vissé à la paroi externe du col.

Ainsi, le bouchon est retiré lors de l'initiation de la phase de prélèvement d'huile, et l'opercule est uniquement déplacée avant l'introduction du tuyau de prélèvement pour minimiser les risques d'introduire des polluants dans le système mécanique.

Selon une possibilité compatible avec les précédentes, le système mécanique peut comprendre une prise de remplissage distincte du dispositif de prélèvement pour introduire l'huile dans le volume interne.

Eventuellement, un plan horizontal passe par l'extrémité externe, ce plan horizontal passant par la prise de remplissage ou étant au-dessus de la prise de remplissage tant que le système mécanique présente un angle de tangage et un angle de roulis compris respectivement dans une plage de tangage prédéterminée et une plage de roulis prédéterminée.

L'obturateur du dispositif de prélèvement est au minimum au même niveau, en altitude, que la prise de remplissage. Cette caractéristique permet d'avoir le même niveau de risque de débordement via la tuyauterie que via la prise de remplissage. Eventuellement, l'obturateur est placé de manière à faciliter la phase de prélèvement d'huile. Par exemple, l'obturateur peut être placé à côté de la prise de remplissage.

Selon un autre aspect, le système mécanique peut être une boîte de transmission de puissance.

Selon un autre aspect, un véhicule peut être muni d'un système mécanique selon l'invention. Par exemple, un giravion peut comprendre au moins un moteur mettant en mouvement un système mécanique selon l'invention, ce système mécanique mettant en mouvement au moins un rotor.

L'invention et ses avantages apparaîtront avec plus de détails dans le cadre de la description qui suit avec des exemples donnés à titre illustratif en référence aux figures annexées qui représentent :
la figure 1, un schéma illustrant un système mécanique selon l'invention,
la figure 2, un schéma illustrant un système mécanique muni d'un dispositif de prélèvement obturé,
la figure 3, un schéma illustrant le système mécanique de la figure 2 avant insertion d'un tuyau de prélèvement,
la figure 4, un schéma illustrant un système mécanique de la figure 2 lors de l'insertion du tuyau de prélèvement, et
la figure 5, un schéma illustrant un système mécanique de la figure 2 lors de l'extraction du tuyau de prélèvement.

Les éléments présents dans plusieurs figures distinctes sont affectés d'une seule et même référence.

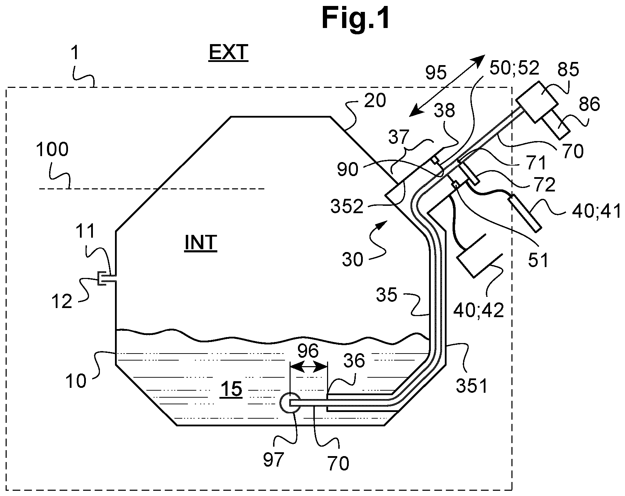

La figure 1 illustre un système mécanique 10 selon l'invention. Le système mécanique 10 est muni d'une enveloppe 20 délimitant un volume interne INT contenant de l'huile 15. L'enveloppe 20 peut comprendre un ou plusieurs carters.

Ce système mécanique 10 peut être agencé au sein d'un véhicule 1. Par exemple, ce véhicule 1 peut être un aéronef, voire un giravion.

Par exemple, le système mécanique 10 peut comprendre au moins un élément mobile non représenté, tel qu'un arbre, un pignon, une roue, des paliers ou autres, lubrifié et/ou refroidi par un système de lubrification et/ou de refroidissement puissant l'huile 15. Par exemple, le système mécanique 10 peut être une boîte de transmission de puissance.

Indépendamment de son agencement, le système mécanique 10 peut comprendre un dispositif de remplissage muni d'une prise de remplissage 11 pour permettre d'introduire l'huile 15 dans le volume interne INT. Le dispositif de remplissage peut comprendre un bouchon de remplissage 12 pour obturer la prise de remplissage 11 et/ou une crépine par exemple.

Indépendamment de ces aspects, le système mécanique 10 comporte un dispositif de prélèvement 30 qui a pour fonction de permettre de prélever au moins une partie de l'huile 15, à des fins d'analyse par exemple. Ce dispositif de prélèvement 30 est distinct du dispositif de remplissage éventuel précédemment décrit.

Le dispositif de prélèvement 30 comprend une tuyauterie 35 fixe par rapport à l'enveloppe 20. La tuyauterie 35 peut être fixée à l'enveloppe 20 voire peut être au moins partiellement ménagée à l'aide d'éléments de l'enveloppe 20.

Cette tuyauterie 35 s'étend d'une extrémité interne 36 située dans le volume interne INT jusqu'à une extrémité externe 38 débouchant sur un milieu extérieur EXT situé à l'extérieur du système mécanique 10. L'extrémité externe 38 peut être située dans le milieu extérieur EXT.

En particulier, l'extrémité externe 38 est présente sur une zone extrémale de la tuyauterie 35 dénommée « col 37 ». Ce col 37 peut être situé au moins partiellement dans le milieu extérieur EXT par exemple.

Selon l'exemple illustré, la tuyauterie 35 comporte un conduit structurel 351 intégré à l'enveloppe 20 débouchant sur une prise de prélèvement 352 fixée à l'enveloppe 20.

Eventuellement, le col 37 peut se situer à la même altitude ou à une altitude supérieure à l'altitude de la prise de remplissage 11. Un plan horizontal 100 passant par l'extrémité externe 38 peut alors soit passer par la prise de remplissage 11 soit passer au-dessus de la prise de remplissage 11 tant que le système mécanique 10 présente un angle de tangage et un angle de roulis compris respectivement dans une plage de tangage prédéterminée et une plage de roulis prédéterminée.

De plus, le dispositif de prélèvement 30 comprend un obturateur 40 manœuvrable par un opérateur pour isoler sur commande l'intérieur de la tuyauterie 35 du milieu extérieur EXT.

L'obturateur 40 peut comporter un opercule 41. L'opercule 41 est mobile d'une position ouverte laissant un accès vers l'intérieur de la tuyauterie 35 jusqu'à une position fermée dans laquelle l'opercule 41 ferme l'extrémité externe 38. Eventuellement, l'opercule peut comprendre un moyen d'étanchéité 80, tel qu'un joint plaqué contre le col lorsque l'opercule ferme la tuyauterie. Alternativement, le col peut comprendre un tel moyen d'étanchéité 80 plaqué contre l'opercule lorsque l'opercule ferme la tuyauterie.

Selon l'exemple de la figure 1, l'opercule 41 peut être vissé à la tuyauterie 35 dans la position fermée. Eventuellement, l'opercule 41 peut être reliée par un lien à la tuyauterie 35 ou à l'enveloppe 20 pour ne pas être perdu dans une position ouverte.

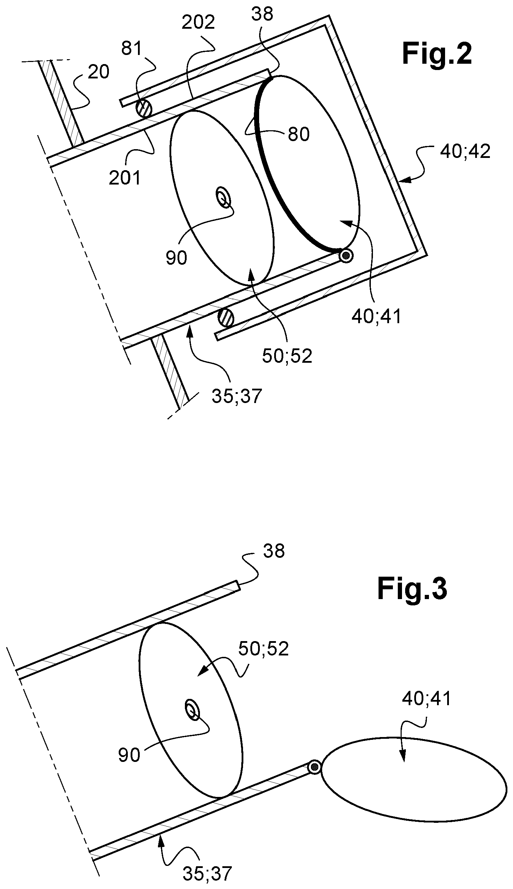

Selon l'exemple des figures 2 à 5 décrites par la suite, l'opercule 41 peut être articulé au col 37 afin d'être mobile en rotation par rapport à la tuyauterie 35.

De manière alternative ou complémentaire et en référence à la figure 1, l'obturateur 40 peut comporter un bouchon 42. Un tel bouchon 42 peut être vissé à une paroi externe 202 du col 37. Eventuellement, un lien peut relier le bouchon 42 à la tuyauterie 35 ou à l'enveloppe 20 pour ne pas perdre le bouchon 42 lorsqu'il est enlevé. Eventuellement, le bouchon peut comprendre un organe d'étanchéité 81, tel qu'un joint plaqué contre le col lorsque le bouchon ferme la tuyauterie. Alternativement, le col peut comprendre un tel organe d'étanchéité 81 plaqué contre le bouchon lorsque le bouchon ferme la tuyauterie.

Selon un autre aspect, le dispositif de prélèvement 30 peut comprendre un tuyau de prélèvement 70 amovible. Ce tuyau de prélèvement 70 peut être inséré dans la tuyauterie 35 lors d'une phase de prélèvement d'huile.

Le tuyau de prélèvement 70 peut faire partie d'un kit comprenant en outre une pompe 85 et/ou un récipient 86. La pompe 85 peut être fixée au tuyau de prélèvement 70 et peut déboucher sur le récipient 86.

Par ailleurs, le tuyau de prélèvement 70 est plus fin que la tuyauterie 35 pour pourvoir être introduit dans cette tuyauterie 35. Par exemple, le tuyau de prélèvement 70 présente un diamètre externe inférieur à un diamètre interne de la tuyauterie 35.

Par ailleurs, le tuyau de prélèvement 70 peut être souple alors que la tuyauterie 35 peut être rigide.

Selon un autre aspect, le tuyau de prélèvement 70 peut avoir une longueur calibrée par rapport à une longueur de la tuyauterie 35. Cette longueur calibrée peut être définie afin que le tuyau de prélèvement 70 sorte de part et d'autre de la tuyauterie 35 sur des distances 95, 96 prédéterminées. Ainsi, le tuyau de prélèvement 70 présente une longueur 96 dans le volume interne INT à la sortie de l'extrémité interne 36, et une longueur de dépassement 95 en dehors de la tuyauterie 35 du côté du milieu extérieur EXT. Lors d'une phase de prélèvement, un opérateur introduit le tuyau de prélèvement 70 dans la tuyauterie 35 en prenant soin d'avoir la longueur de dépassement 95 souhaitée. Cette caractéristique permet de garantir que le tuyau de prélèvement 70 atteint la zone de prélèvement souhaitée 97 dans le volume interne INT.

De manière complémentaire ou alternative, le tuyau de prélèvement 70 a un marquage 71, tel qu'un trait coloré par exemple. L'opérateur peut déplacer le tuyau de prélèvement 70 par rapport à la tuyauterie 35 de manière à placer le marquage 71 dans une position prédéterminée par rapport à la tuyauterie 35, par exemple au niveau de l'extrémité externe 38 selon l'exemple illustré.

De manière complémentaire ou alternative, le tuyau de prélèvement 70 a une protubérance 72 formant une butée. Une telle protubérance 72 peut à la fois permettre de positionner correctement le tuyau de prélèvement 70, et d'empêcher éventuellement ce tuyau de prélèvement 70 de tomber dans la tuyauterie 35, et par suite dans le système mécanique 10.

Selon un autre aspect, le dispositif de prélèvement 30 comprend une barrière anti-polluant 50 agencée dans le col 37 pour limiter les risques de polluer le volume interne INT notamment lors d'une phase de prélèvement d'huile. La barrière anti-polluant 50 restreint une surface de passage interne de la tuyauterie 35 à un passage 90 apte à être traversé par le tuyau de prélèvement 70.

Cette barrière anti-polluant 50 peut être disposée dans la tuyauterie 35 entre l'extrémité externe 38 et l'extrémité interne 36.

La barrière anti-polluant 50 peut comporter une membrane élastique 52 qui est disposée dans la tuyauterie 35, et en particulier dans le col 37.

Cette membrane élastique 52 peut être amovible. Par exemple, la membrane élastique 52 est solidaire d'un anneau 51 de la barrière anti-polluant 50. Cet anneau 51 peut être vissé à une paroi interne du col 37. Selon un autre exemple, cet anneau 51 peut comprendre des ergots radiaux glissant dans des glissières de la tuyauterie 35.

Selon un autre exemple la membrane élastique 52 est solidarisée à la tuyauterie 35, par collage par exemple.

Indépendamment de sa réalisation, la barrière anti-polluant 50 s'étend à partir de la tuyauterie 35 et délimite le passage 90 restreint.

Autrement dit, la barrière anti-polluant 50 est solidarisée au col 37 afin que la membrane élastique 52 réduise une surface de passage au sein de la tuyauterie 35 au seul passage 90. Par exemple, la membrane élastique 52 a une forme annulaire, ou une forme d'un disque présentant une découpure formant ledit passage 90. Eventuellement, le passage 90 ménagé peut avoir des dimensions plus petites que le tuyau de prélèvement 70.

Eventuellement, la membrane élastique 52 est obtenue avec une matière du groupe des élastomères.

Les figures 2 à 5 illustrent le fonctionnement de l'invention avec un obturateur ayant un bouchon et un opercule. Un fonctionnement similaire est de fait obtenu en présence uniquement du bouchon ou de l'opercule, les opérations liées au bouchon ou à l'opercule étant identiques aux opérations qui suivent.

Au repos et tel qu'illustré sur la figure 2, l'obturateur 40 empêche des polluants d'atteindre la membrane élastique 52. Selon l'exemple illustré, l'obturateur 40 comporte un opercule 41 et un bouchon 42. L'opercule 41 est dans une position fermée et recouvre l'extrémité externe 38. De plus, le bouchon 42 est vissé au col 37.

Pour effectuer un prélèvement d'huile et tel qu'illustré sur la figure 3, un opérateur manœuvre l'obturateur 40. Selon l'exemple donné, l'opérateur dévisse le bouchon 42. L'opérateur manœuvre ensuite l'opercule 41 pour le positionner dans une position ouverte juste avant d'introduire le tuyau de prélèvement 70. Une fois l'opercule 41 déplacé dans sa position ouverte, la membrane élastique 52 limite les risques de pénétration de polluants dans la tuyauterie 35 et le système mécanique 10 puisque la surface de passage dans le col 37 est réduite à l'aire du passage 90.

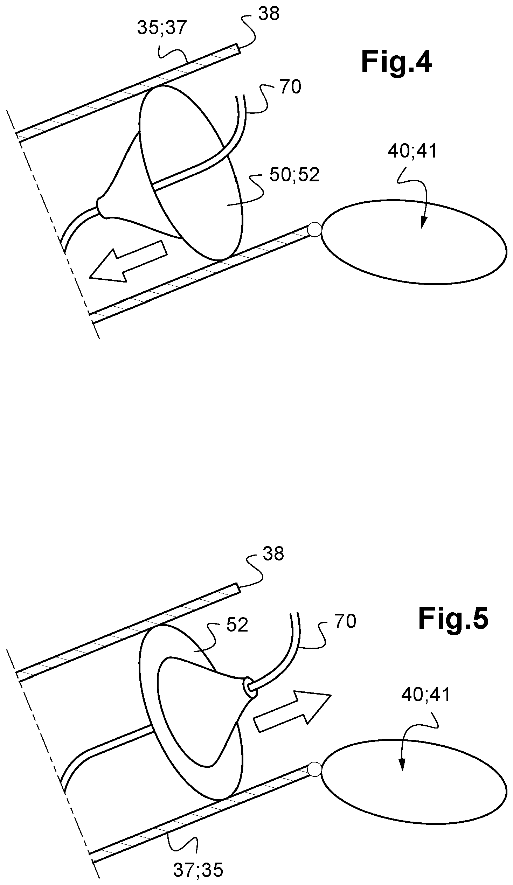

Tel qu'illustré sur la figure 4, l'opérateur introduit alors le tuyau de prélèvement 70. Le tuyau de prélèvement 70 déforme la membrane élastique 52 pour agrandir le passage 90. Le tuyau de prélèvement 70 traverse alors la membrane élastique 52. De plus, la membrane élastique 70 épouse la forme du tuyau de prélèvement 70 qui laisse peu voire aucun espace entre la membrane élastique 52 et le tuyau de prélèvement 70. La membrane élastique 52 peut aussi empêcher le tuyau de prélèvement 70 de tomber dans la tuyauterie 35 et, par suite, dans le système mécanique 10.

L'opérateur peut alors positionner de la manière adéquate le tuyau de prélèvement 70 par rapport à la tuyauterie, par exemple à l'aide du marquage et/ou de la protubérance précités. L'opérateur peut le cas échéant actionner la pompe 85 connectée au tuyau de prélèvement 70 pour prélever l'huile 15 requise.

A l'issue du prélèvement et tel qu'illustré sur la figure 5, l'opérateur peut alors tirer sur le tuyau de prélèvement 70 pour l'extraire en dehors de la tuyauterie 35. L'opérateur repositionne l'opercule 41 dans sa position fermée et visse le bouchon 42 à la tuyauterie.

Naturellement, la présente invention est sujette à de nombreuses variations quant à sa mise en œuvre. Bien que plusieurs modes de réalisation aient été décrits, on comprend bien qu'il n'est pas concevable d'identifier de manière exhaustive tous les modes possibles. L'invention est définie par les revendications.

## Revendications

1. Système mécanique (10) muni d'une enveloppe (20) délimitant un volume interne (INT) contenant de l'huile (15),
**caractérisé en ce que** le système mécanique (10) comporte un dispositif de prélèvement (30) pour prélever au moins une partie de l'huile (15), le dispositif de prélèvement (30) comprenant au moins une tuyauterie (35) solidaire de l'enveloppe (20), la tuyauterie (35) s'étendant d'une extrémité interne (36) située dans le volume interne (INT) jusqu'à un col (37) comprenant une extrémité externe (38) ouverte sur un milieu extérieur (EXT) situé à l'extérieur du système mécanique (10), le dispositif de prélèvement (30) comprenant un obturateur (40) manœuvrable par un opérateur pour obturer le col (37), le dispositif de prélèvement (30) comprenant dans le col (37) une barrière anti-polluant (50) ménageant un passage (90) restreint apte à être traversé par un tuyau de prélèvement (70).

2. Système mécanique selon la revendication 1,
**caractérisé en ce que** ladite barrière anti-polluant (50) est disposée dans la tuyauterie (35) entre l'extrémité externe (38) et l'extrémité interne (36).

3. Système mécanique selon l'une quelconque des revendications 1 à 2,
**caractérisé en ce que** ladite barrière anti-polluant (50) est amovible.

4. Système mécanique selon l'une quelconque des revendications 1 à 3,
**caractérisé en ce que** ladite barrière anti-polluant (50) comporte une membrane élastique (52) délimitant ledit passage (90).

5. Système mécanique selon la revendication 4,
**caractérisé en ce que** la membrane élastique (52) comporte une matière du groupe des élastomères.

6. Système mécanique selon l'une quelconque des revendications 4 à 5,
**caractérisé en ce que** le dispositif de prélèvement (30) comporte le tuyau de prélèvement (70), la membrane élastique (52) s'étirant lorsque le tuyau de prélèvement (70) est introduit en élargissant ledit passage (90), la membrane élastique (52) épousant le tuyau de prélèvement (70).

7. Système mécanique selon l'une quelconque des revendications 1 à 6,
**caractérisé en ce que** le dispositif de prélèvement (30) comporte le tuyau de prélèvement (70), le tuyau de prélèvement (70) étant plus fin que la tuyauterie (35) pour pourvoir être introduit dans la tuyauterie (35) et passer au travers de ladite barrière anti-polluant (50) via le passage (90).

8. Système mécanique selon l'une quelconque des revendications 1 à 7,
**caractérisé en ce que** le dispositif de prélèvement (30) comporte le tuyau de prélèvement (70), le tuyau de prélèvement (70) a une longueur calibrée par rapport à une longueur de la tuyauterie (35) afin de sortir de part et d'autre de la tuyauterie (35) et en particulier en dehors de la tuyauterie (35) du côté du milieu extérieur (EXT) d'une longueur de dépassement (95) prédéterminée durant une phase de prélèvement d'huile.

9. Système mécanique selon l'une quelconque des revendications 1 à 8,
**caractérisé en ce que** le dispositif de prélèvement (30) comporte le tuyau de prélèvement (70), le tuyau de prélèvement (70) a au moins un marquage (71) ou une protubérance (72) configuré pour placer le tuyau de prélèvement (70) à une position prédéterminée par rapport à la tuyauterie (35).

10. Système mécanique selon l'une quelconque des revendications 1 à 9,
**caractérisé en ce que** l'obturateur (40) comporte un opercule (41) articulé au col (37) ou vissé au col (37) pour obturer ce col (37).

11. Système mécanique selon l'une quelconque des revendications 1 à 10,
**caractérisé en ce que** l'obturateur (40) comporte un bouchon (42) vissé à une paroi externe (202) du col (37).

12. Système mécanique selon les revendications 10 et 11, **caractérisé en ce que** le bouchon (42) recouvre l'opercule (41) quand ce bouchon est vissé à la paroi externe (202) du col (37).

13. Système mécanique selon l'une quelconque des revendications 1 à 12,
**caractérisé en ce que** le système mécanique (10) comprend une prise de remplissage (11) distincte du dispositif de prélèvement (30) pour introduire l'huile (15) dans le volume interne (INT), un plan horizontal (100) passant par l'extrémité externe (38), ce plan horizontal (100) passant par la prise de remplissage (11) ou étant au-dessus de la prise de remplissage (11) tant que le système mécanique (10) présente un angle de tangage et un angle de roulis compris respectivement dans une plage de tangage prédéterminée et une plage de roulis prédéterminée.

14. Véhicule (1) muni d'un système mécanique (10),
**caractérisé en ce que** le système mécanique (10) est selon l'une quelconque des revendications 1 à 13.

## Patentansprüche

1. Mechanisches System (10) mit einem Gehäuse (20), das einen Innenraum (INT) begrenzt, der Öl (15) enthält,
**dadurch gekennzeichnet, dass** das mechanische System (10) eine Entnahmevorrichtung (30) zum Entnehmen mindestens eines Teils des Öls (15) umfasst, die Entnahmevorrichtung (30) mindestens eine fest mit dem Gehäuse (20) verbundene Rohrleitung (35) umfasst, die Rohrleitung (35) sich von einem inneren Ende (36), das sich im Innenraum (INT) befindet, bis zu einem Hals (37) erstreckt, der ein äußeres Ende (38) aufweist, das zu einer außerhalb des mechanischen Systems (10) befindlichen äußeren Umgebung (EXT) hin offen ist, die Entnahmevorrichtung (30) einen von einem Bediener betätigbaren Verschluss (40) zum Verschließen des Halses (37) umfasst, und die Entnahmevorrichtung (30) im Hals (37) eine Schmutzbarriere (50) umfasst, die einen verengten Durchgang (90) bildet, der eingerichtet ist, um von einem Entnahmeschlauch (70) durchquert zu werden.

2. Mechanisches System nach Anspruch 1,
**dadurch gekennzeichnet, dass** die Schmutzbarriere (50) in der Rohrleitung (35) zwischen dem äußeren Ende (38) und dem inneren Ende (36) angeordnet ist.

3. Mechanisches System nach einem der Ansprüche 1 bis 2,
**dadurch gekennzeichnet, dass** die Schmutzbarriere (50) abnehmbar ist.

4. Mechanisches System nach einem der Ansprüche 1 bis 3,
**dadurch gekennzeichnet, dass** die Schmutzbarriere (50) eine elastische Membran (52) aufweist, die den Durchgang (90) begrenzt.

5. Mechanisches System nach Anspruch 4,
**dadurch gekennzeichnet, dass** die elastische Membran (52) ein Material aus der Gruppe der Elastomere umfasst.

6. Mechanisches System nach einem der Ansprüche 4 bis 5,
**dadurch gekennzeichnet, dass** die Entnahmevorrichtung (30) den Entnahmeschlauch (70) umfasst, die elastische Membran (52) sich beim Einführen des Entnahmeschlauchs (70) dehnt und dabei den Durchgang (90) erweitert, und die elastische Membran (52) sich an den Entnahmeschlauch (70) anschmiegt.

7. Mechanisches System nach einem der Ansprüche 1 bis 6,
**dadurch gekennzeichnet, dass** die Entnahmevorrichtung (30) den Entnahmeschlauch (70) umfasst, der Entnahmeschlauch (70) dünner ist als die Rohrleitung (35), um in die Rohrleitung (35) eingeführt werden zu können und über den Durchgang (90) durch die Schmutzbarriere (50) hindurchzutreten.

8. Mechanisches System nach einem der Ansprüche 1 bis 7,
**dadurch gekennzeichnet, dass** die Entnahmevorrichtung (30) den Entnahmeschlauch (70) umfasst, und der Entnahmeschlauch (70) eine im Verhältnis zu einer Länge der Rohrleitung (35) kalibrierte Länge hat, um während einer Ölentnahmephase beidseitig aus der Rohrleitung (35) überzustehen und insbesondere auf der Seite der Außenumgebung (EXT) um eine vorgegebene Überstandslänge (95) aus der Rohrleitung (35) überzustehen.

9. Mechanisches System nach einem der Ansprüche 1 bis 8,
**dadurch gekennzeichnet, dass** die Entnahmevorrichtung (30) den Entnahmeschlauch (70) umfasst, und der Entnahmeschlauch (70) mindestens eine Markierung (71) oder einen Vorsprung (72) aufweist, die bzw. der konfiguriert ist, um den Entnahmeschlauch (70) relativ zur Rohrleitung (35) in einer vorgegebenen Position zu platzieren.

10. Mechanisches System nach einem der Ansprüche 1 bis 9,
**dadurch gekennzeichnet, dass** der Verschluss (40) einen Deckel (41) aufweist, der am Hals (37) angelenkt oder mit dem Hals (37) verschraubt ist, um diesen Hals (37) zu verschließen.

11. Mechanisches System nach einem der Ansprüche 1 bis 10,
**dadurch gekennzeichnet, dass** der Verschluss (40) einen Stopfen (42) aufweist, der auf eine Außenwand (202) des Halses (37) geschraubt ist.

12. Mechanisches System nach den Ansprüchen 10 und 11,
**dadurch gekennzeichnet, dass** der Stopfen (42) den Deckel (41) überdeckt, wenn der Stopfen auf die Außenwand (202) des Halses (37) geschraubt ist.

13. Mechanisches System nach einem der Ansprüche 1 bis 12,
**dadurch gekennzeichnet, dass** das mechanische System (10) einen von der Entnahmevorrichtung (30) getrennten Einfüllstutzen (11) zum Einfüllen des Öls (15) in den Innenraum (INT) umfasst, wobei eine horizontale Ebene (100) durch das äußere Ende (38) verläuft, wobei diese horizontale Ebene (100) durch den Einfüllstutzen (11) verläuft oder sich über dem Einfüllstutzen (11) befindet, solange das mechanische System (10) einen Nickwinkel und einen Rollwinkel aufweist, die jeweils in einem vorgegebenen Nickwinkelbereich und einem vorgegebenen Rollwinkelbereich liegen.

14. Verkehrsmittel (1) mit einem mechanischen System (10),
**dadurch gekennzeichnet, dass** das mechanische System (10) gemäß einem beliebigen der Ansprüche 1 bis 13 ist.

## Claims

1. Mechanical system (10) provided with an envelope (20) delimiting an internal volume (INT) containing oil (15),
**characterised in that** the mechanical system (10) has a removal device (30) to remove at least some of the oil (15), the removal device (30) comprising at least one pipe (35) secured to the envelope (20), the pipe (35) extending from an internal end (36) located in the internal volume (INT) to a neck (37) comprising an external end (38) open on an external environment (EXT) located outside of the mechanical system (10), the removal device (30) comprising a blocker (40) which can be set in motion by an operator to block the neck (37), the removal device (30) comprising in the neck (37), an anti-polluting barrier (50) making a restricted passage (90) capable of being passed through by a removal pipe (70).

2. Mechanical system according to claim 1,
**characterised in that** said anti-polluting barrier (50) is disposed in the pipe (35) between the external end (38) and the internal end (36).

3. Mechanical system according to any one of claims 1 to 2,
**characterised in that** said anti-polluting barrier (50) is removable.

4. Mechanical system according to any one of claims 1 to 3,
**characterised in that** said anti-polluting barrier (50) has an elastic membrane (52) delimiting said passage (90).

5. Mechanical system according to claim 4,
**characterised in that** the elastic membrane (52) has a material from the elastomer group.

6. Mechanical system according to any one of claims 4 to 5,
**characterised in that** the removal device (30) has a removal pipe (70), the elastic membrane (52) being stretched when the removal pipe (70) is introduced by expanding said passage (90), the elastic membrane (52) moulding the removal pipe (70).

7. Mechanical system according to any one of claims 1 to 6,
**characterised in that** the removal device (30) has the removal pipe (70), the removal pipe (70) being thinner than the pipe (35) to be able to be inserted in the pipe (35) and to pass through said anti-polluting barrier (50) via the passage (90).

8. Mechanical system according to any one of claims 1 to 7,
**characterised in that** the removal device (30) has the removal pipe (70), the removal pipe (70) has a length calibrated with respect to a length of the pipe (35), in order to exit on either side of the pipe (35), and in particular, outside of the pipe (35) on the side of the external environment (EXT) of a predetermined overrun length (95) during an oil removal phase.

9. Mechanical system according to any one of claims 1 to 8,
**characterised in that** the removal device (30) has the removal pipe (70), the removal pipe (70) has at least one marking (71) or a protrusion (72) configured to place the removal pipe (70) at a predetermined position with respect to the pipe (35).

10. Mechanical system according to any one of claims 1 to 9,
**characterised in that** the blocker (40) has a lid (41) articulated to the neck (37) or screwed to the neck (37) to block this neck (37).

11. Mechanical system according to any one of claims 1 to 10,
**characterised in that** the blocker (40) has a stopper (42) screwed to an external wall (202) of the neck (37).

12. Mechanical system according to claims 10 and 11,
**characterised in that** the stopper (42) covers the lid (41) when this stopper is screwed to the external wall (202) of the neck (37).

13. Mechanical system according to any one of claims 1 to 12,
**characterised in that** the mechanical system (10) comprises a filling inlet (11) distinct from the removal device (30) to insert the oil (15) in the internal volume (INT), a horizontal plane (100) passing through the external end (38), the horizontal plane (100) passing through the filling inlet (11), or being above the filling inlet (11), as the mechanical system (10) has a pitch angle and a roll angle comprised respectively in a predetermined pitch range and a predetermined roll range.

14. Vehicle (1), provided with a mechanical system (10),
**characterised in that** the mechanical system (10) is according to any one of claims 1 to 13.
